# EUROPEAN PATENT SPECIFICATION

(11) **EP 2 822 536 B1**
(45) Date of publication and mention of the grant of the patent: **20.04.2016**
(21) Application number: 13715448.0
(22) Date of filing: 04.02.2013
(51) Int. Cl.: A61K 9/00

(54) **TRANSDERMAL ADMINISTRATION OF PROSTAGLANDIN E1 FOR THE TREATMENT OF OCULAR ISCHEMIA**
TRANSDERMALE VERABREICHUNG VON PROSTAGLANDIN E1 ZUR BEHANDLUNG VON OKULÄRER ISCHÄMIE
ADMINISTRATION TRANSDERMIQUE DE PROSTAGLANDINE E1 POUR LE TRAITEMENT DE L'ISCHÉMIE OCULAIRE

(30) Priority: 02.02.2012 IT RM20120036
(43) Date of publication of application: 14.01.2015
(73) Proprietor: Steigerwalt, Robert, Davis Jr., I-00195 Roma (IT)
(72) Inventor: Steigerwalt, Robert, Davis Jr., I-00195 Roma (IT)
(74) Representative: Banchetti, Marina
(86) International application number: PCT/IT2013/000032
(87) International publication number: WO 2013/114418

(56) References cited:
- WO-A1-2008/041054
- WO-A1-2011/095938
- US-A- 5 380 760
- US-A- 5 849 792
- Akiharu Isowaki ET AL: "Drug Delivery to the Eye with a Transdermal Therapeutic System", Biol. Pharm. Bull., 1 January 2003 (2003-01-01), pages 69-72, XP055035905, Retrieved from the Internet: URL:https://www.jstage.jst.go.jp/article/b pb/26/1/26_1_69/_pdf [retrieved on 2012-08-21]

## Description

### Field of the invention

The present invention concerns the use of prostaglandin E₁ in trans-dermal application for the treatment of ocular ischemia. More specifically, the invention concerns the use of a preparation containing prostaglandin E₁ in a vehicle suitable for administration on the skin, for the treatment by transdermal route of chronic ophthalmic diseases of an ischemic nature and, in general, for the treatment of ophthalmic diseases where an ischemic mechanism of the ocular district is involved.

### Background of the invention

In the ophthalmology field, the generic term **optic neuropathy** denotes forms of optic nerve impairment, which can be, in general, of an inflammatory nature such as the inflammation of the optic nerve which is referred to by the specific term optic neuritis, or they may be of a traumatic or toxic origin, or also they may be originated by compression. The most important forms of optic neuropathy are however the ischemic forms, which are due to an insufficient blood flow to the optic nerve, resulting in an inadequate supply of oxygen to neuronal cells which may cause their death, with consequent total or partial loss of vision.

**Ischemic optic neuropathy** is called **"anterior"** or **"posterior"** according to whether the affected tissues are those of the head of the optic nerve (optic disc) or those of the retro-bulbar optic nerve portion. The more common **anterior ischemic optic neuropathy** is caused by an infarction of the optic nerve head, and represents the most common cause of acute deficit of visual function in patients over 50 years of age. It is characterized by a severe reduction in visual acuity associated with visual field deficiencies, ischemic edema of the optic nerve head and slight peripapillary hemorrhage signs. The disease may be differentiated in two different clinical forms: the **non-arteritic** form, more common, which is caused by an occlusion on atherosclerotic basis associated with local risk factors linked to the shape of the optic nerve head, and the **arteritic** form, which is part of a generalized disease (Horton's arteritis), originated by an inflammatory-based occlusion. The outcome of the ischemic event is, in both forms, the atrophy of nerve fibers and the appearance of optical disc paleness.

**Arteritic anterior ischemic optic neuropathy** (AAION) is an inflammatory affection of the optic nerve that typically affects people over 65 years of age and preferentially affects the medium-sized arteries, in particular the superficial temporal artery, the ophthalmic artery, the posterior ciliary arteries. It presents with a serious and sudden unilateral visual impairment, associated with pain around the eyes, and a generalized headache, fever, weight loss associated with anemia, difficulty in chewing, accompanied by a tortuous, painful and pulsating temporal artery. Within a few months the edema resolves but a severe optic atrophy remains, resulting in a significant deficit in the visual function.

**Non-arteritic anterior ischemic optic neuropathy** (NAION) is a primitive inflammatory form of the optic nerve that can occur as an isolated event in subjects aged between 45 and 65 years, and is usually due to blockage of the short posterior ciliary arteries. It usually presents with an acute onset visual impairment, without pain or prior warning symptoms. The optic disc edema gradually resolves and an optic atrophy remains, associated with further thinning of the retinal arteries.

**Posterior ischemic option neuropathy** (PION), more rare but even more devastating, is characterized by an ischemic damage to the retro-bulbar portion of the optic nerve, and may also have an **arteritic** form (A-PION), caused by a generalized inflammatory disease that is considered autoimmune in origin (Horton's arteritis) and a **non-arteritic** form, which is the one of higher incidence between the two.

**Posterior arteritic ischemic optic neuropathy** occurs especially in aged women, with a sudden and painless loss of visual function, which in most cases involves the perception of colors. The deficit is initially unilateral, but in the absence of an immediate anti-inflammatory treatment, it quickly evolves to involve both eyes, causing a loss of visual function. The treatment is intended to prevent the further progression of the disease, but the visual loss is irreversible.

The **non-arteritic** form of PION can hit, with a mechanism which is likely to be multifactorial, patients who have undergone long (not ophthalmic) surgery, or surgery associated with significant blood loss, or in patients who have had severe bleeding, such as after accidents. For this reason such form is referred to as **perioperative posterior ischemic optic neuropathy,** or shock-induced posterior ischemic optic neuropathy. In this case the loss of visual function is often discovered by the patient on awakening from anesthesia.

Other forms of acute ophthalmic pathologies of ischemic nature affect the retina, such as **retinal vascular occlusions,** which consist of an interruption of arterial or venous blood flow in the retina (thrombosis). The main symptom is a severe and sudden vision reduction, usually painless. The condition usually affects one eye only, and the causes are to be found in alterations of the vessels walls caused by high blood pressure, atherosclerosis and formation of thrombi or emboli.

The **retinal arterial occlusions** affect the central retinal artery (central retinal arterial occlusion) or one of its arterial vascular branches that are directed towards the peripheral retinal districts (retinal arterial occlusion of branch). They occur with an acute and dramatic event, i.e. the spastic or embolic closing of the arterial blood flow, which results in a loss of vision that in a few hours becomes severe and unrecoverable, especially if the central retinal artery is affected. If a peripheral branch is affected, the visual loss is more sectoral and central vision can be preserved. In practice, an infarction occurs in the retina, and therefore the anatomical damage and reduction of vision are serious, and often definitive. Treatment should be immediate to try to solve the occlusion before the development of irreversible damage due to ischemia; the prognosis is, however, reserved even in early treated cases.

**Retinal venous occlusions** are events much more frequent than arterial occlusions, and have a generally better prognosis. They occur when the occlusion affect the central retinal vein (central retinal vein occlusion) or one of its branches (branch retinal vein occlusion), and can be ischemic or non-ischemic. The obstacle to the venous blood flow determines the consequent blood spillage, edema and substances of various kinds leaking from the vessel itself, and the degree of occlusion determined by the thrombus characterizes two different forms of occlusion: the edematous form (partial occlusion of the vein) and the ischemic form (total occlusion of the vein). The symptoms, proportional to the severity of the vessel occlusion, are represented by a variable sudden decrease of the vision without any pain.

As already noted, all the ophthalmic acute pathologies cited above need to be promptly treated in order to save at least partially the vision, and prevent the progression of the disease toward the total loss of visual capacity. A therapy to date practiced with success in many cases of ischemic ocular pathologies is the intravenous administration of prostaglandins, in particular prostaglandin E₁ (PGE₁) (Steigerwalt RD Jr et al., Arteritic anterior ischemic optic neuropathy treated with intravenous prostaglandin E1 and steroids. Int J Angiol. 2010; 19:113-5; Masayuki K et al., A case of recurrent anterior ischemic optic neuropathy simulating optic neuritis; Jap J Clin Ophthalm 2009; 57:8 1381-6; Steigerwalt RD Jr et al., Nonarteritic anterior ischemic optic neuropathy treated with intravenous prostaglandin E1 and steroids. Int J Angiol. 2008; 17:193-63; Steigerwalt RD et al., Non-arteritic posterior ischaemic optic neuropathy treated with intravenous prostaglandin E1 and oral corticosteroids. Neuro-ophthalmology. 2011; 35:81-4; Steigerwalt RD Jr et al., Acute branch retinal arterial embolism successfully treated with intravenous prostaglandin E1-Case Reports. Angiology. 2003; 54(4):491-3).

Prostaglandins are a family of biologically active substances ubiqui-tously produced in the body, with extremely complex and sometimes conflicting biological functions, including stimulation of smooth muscle, dilation of small arteries and bronchi, lowering of blood pressure, inhibition of gastric secretion, promotion of platelet aggregation. Their general structure is seen as deriving form a hypothetical basic structure, prostanoic acid, a fatty acid having twenty carbon atoms characterized by a five-membered ring linked to two linear side chains with seven and eight carbon atoms (20 C in total), named respectively α chain (head chain, upstream of the ring) and ω chain (tail chain, downstream of the ring).

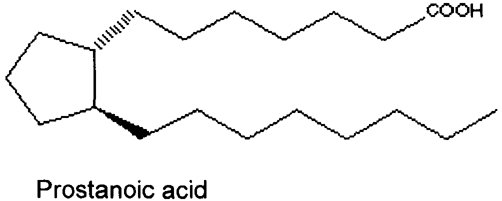

The various subclasses of prostaglandins (PG) differ from each other in the specific features of the five-membered ring, and are identified by a capital letter of the Latin alphabet (A to I), as illustrated by way of example below.

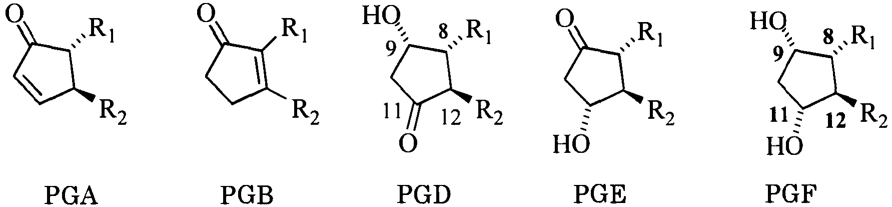

The degree of saturation, in terms of double bonds present total in the two side chains, is indicated by an Arabic numeral subscript in the symbol of the subclass, according to the following scheme:

| | R₁ (α chain) | R₂ (ω chain) |
|---|---|---|
| Series 1 | 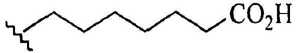 | 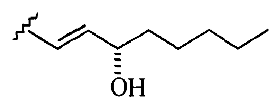 |
| Series 2 | 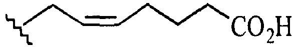 | 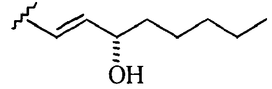 |
| Series 3 | 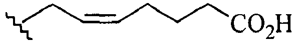 | 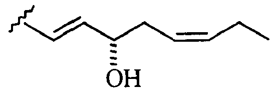 |

According to the above symbology, the prostaglandin E named PGE₁ has the following structural formula:

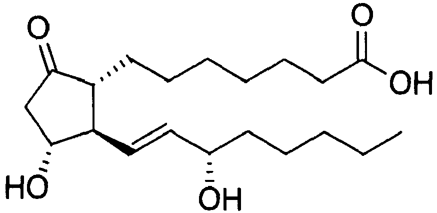

Being powerful vasodilators, prostaglandins E, and specifically PGE₁, have been proposed in literature for the treatment of peripheral occlusive pathologies, of acute myocardial infarction, angina pectoris, gastrointestinal ulcers, etc.. With regard to the ocular ischemic pathologies mentioned above and other ischemic syndromes of the optic nerve, the known literature reports as experienced administration route only the intravenous route, as already mentioned, while for another prostaglandin-type compound with similar potential application, unoprostone (a synthetic derivative of PGF), the experimental literature reports topical ocular administration (Tamaki Y et al, J Ocul Pharmacol Ther. 2001; 17:517-27) and subconjunctival injection (Sugiyama T, et al. Arch Ophthalmol. 2009; 127:454-9). In any case, PGE₁ could not be administered orally, because it is unstable in an acid environment, and is rapidly degraded before it can be absorbed.

With the use of intravenous PGE₁ according to the cited scientific literature the acute ischemic ophthalmic pathologies considered above can normally be resolved, arresting their progression. For this purpose, generally one or two intravenous injection treatment sessions are required.

**Ischemic optic neuropathies and retinopathies of a chronic nature,** on the other hand, can occur in **high myopia,** in **macular degeneration,** in **glaucoma** and in **diabetic retinopathy.**

In **high myopia,** the blood flow may be reduced, and it is more so the higher is the myopia (Karczewicy D, Modrzejewska M. Blood flow in eye arteries assessed by Doppler ultrasound in patients with myopia. Klin Oczna. 2004; 106(1-2 suppl):211-3; Shimada N et al., Reduction of retinal blood flow in high myopia. Graefes Arch Exp Ophthalmol. 2004 April; 242(4):284-8). It has also been ascertained that in high myopia the choroid, which is the tissue of the eye with the greatest blood flow, is thinner than in a healthy eye, and that the choroid thickness is more reduced the higher the myopia is (Fujiwara T et al., Enhanced depth imaging optical coherence tomography of the choroid in highly myopic eyes. Am J Ophthalmol. 2009; 148:445-50).

Since the blood that perfuses the choroid is responsible for the nourishment of the photoreceptor cells of the outer retina, when the choroid is thinner the outer retina receives a lower blood flow, and this causes a lowering of the central vision and a narrowing of the visual field. In high myopia cases, therefore, a total loss of visual functionality is a possible risk. Also in this case, the only treatment proposed which offers appreciable results is the therapy with prostaglandin E₁ by intravenous administration (Steigerwalt RD Jr. et al., Ocular Ischemia in High Myopia Treated with Intravenous Prostaglandin E1. Retinal Cases & Brief Reports. 2009; 3:379-382).

In **age-related macular degeneration** (AMD) there is a gradual aging of the retinal tissues. Chronic ischemia is not the only cause of macular degeneration, but it contributes to the worsening thereof. Various studies on choroidal blood flow showed a lowering of the flow in patients with macular degeneration, with a greater reduction the more advanced the macular degeneration is. Thus, a treatment to increase the choroidal blood flow would be important for the therapy and prophylaxis of age-related macular degeneration as well (Grunwald JE et al., Foveolar choroidal blood flow in age-related macular degeneration. Invest Ophthalmol Vis Sci. 1998;39:385-90; Grunwald JE et al. Reduced foveolar choroidal blood flow in eyes with increasing AMD severity. Invest Ophthalmol Vis Sci. 2005;46:1033-8; Berenberg TL et al., The association between drusen extent and foveolar choroidal blood flow in age-related macular degeneration. Retina. 2011, Aug.).

As is known, **glaucoma** represents the most dangerous and widespread chronic optical neuropathy, and actually encompasses various forms, all linked by the presence of a chronic and progressive damage of the optic nerve that leads, over time, to a gradual and irreversible narrowing of the visual field. An elevated intraocular pressure is the most important risk factor for this disease, but also in this case, ischemia of the optic nerve can have a decisive role for the worsening of the disease, particularly in the low-pressure glaucoma (Flammer J et al., The impact of ocular blood flow in glaucoma. Prog retin Eye Res. 2002; 21:359-93; Zeitz O et al., Glaucoma progression is associated with decreased blood flow velocities in the short posterior ciliary artery. Br J Ophthalmol. 2006; 90:1245-8).

Another chronic ophthalmic disease that is often associated with ischemic problems at the ocular district level is **diabetic retinopathy.** Subjects with diabetes generally develop, as the disease progresses, damage to the retina and other eye structures, due to the fragility of the blood vessels of the microcirculation. Such damage results in lack of an adequate blood supply (hence, oxygen supply) to certain areas of the retina, whose tissues become ischemic and, before dying, release a growth factor for new vessels. In the **proliferative** form of diabetic retinopathy (considered to be the most severe) there is an intense vascular proliferation, with extremely fragile vessels that very often tend to rupture, causing a real blindness, while in the **non-proliferative** form the proliferation of new vessels is absent, and only micro-aneurysms occur, affecting both retinal vessels and those of larger caliber, while exudates and deposits are produced, which equally worsen the vision. In later stages of diabetic retinopathy, therefore, ischemic problems arise, with a lowering of blood flow to the eye (Burgansky-Eliash Z, et al., Reduced retinal blood flow velocity in diabetic retinopathy. Retina. 2010; 30:765-73)

From the foregoing it is evident that also in several chronic ophthalmic pathologies ocular ischemia, if not properly and promptly treated, can lead to a severe damage of the vision. Also in this case, to treat the ischemic event of each of these pathologies, vasodilators are available for systemic administration, and the treatment with intravenous prostaglandin E₁ is feasible. However, a major drawback of such approach lies in the fact that in this case the therapy would be chronic, and then intravenous injection should be practiced repeatedly, probably indefinitely. In the previously mentioned case of therapy of high myopia with intravenously administered PGE₁, for example, the conclusion drawn in the cited publication was that the effect of the intravenous administration lasted no more than 6-8 weeks, and therefore the treatment should be repeated with a corresponding cadence, for extended periods of time.

In general, for all forms of non-acute ocular ischemia, a treatment with prostaglandin E₁ should be carried out intravenously with a cadence variable in the range from 7 to 30 days, for unlimited periods of time. Currently, intravenously administered PGE₁ is the treatment which allows to bring the highest blood flow to the eye tissues the with the lowest side effects. However, intravenous treatments for long periods of time are very challenging for patients, and critically impair the success of the therapy.

Furthermore, it is also to be taken into account that active vasodilators for oral administration existing today, which are sufficiently strong to cause an increase in blood flow in the eye, may cause an excessive lowering of blood pressure and thus give rise to systemic hypotension.

For some therapeutic applications, prostaglandin E₁ has also been proposed in the prior art in delivery forms for transdermal administration, as for example in the patents U.S. 5464868 and U.S. 5681850, both in the name Frölich et al.. These patents disclose compositions based on specific ester derivatives of PGE₁, which would be converted into the desired PGE₁ in the passage through the skin. The therapeutic indications of the proposed composition range from ischemic heart disease and brain ischemia to impotence and from allergic reactions to skin ulcers.

As a further example of transdermal administration of prostaglandin E₁, the European patent EP 0729538**,** in the name of Minnesota Mining and Manufacturing Company (3M), discloses a dosage form for transdermal administration (transdermal patch) comprising (i) an effective amount of PGE₁ or a pharmaceutically acceptable salt or lower alkyl ester thereof, (ii) optionally an excipient with functions of transdermal permeation enhancer selected from isopropyl myristate, ethyl oleate, and a mixture thereof, and (iii) a pressure-sensitive poly-isobutylene adhesive, in which the first two components are dissolved or dispersed substantially uniformly in the poly-isobutylene adhesive. Also in this case, while the dosage form is widely detailed, the possible therapeutic indications of the transdermal patch described are only generically defined as "any condition susceptible of treatment with a prostaglandin"

Along the same lines, the international patent applications publ. No. WO 00/69 469**,** WO01/74279 and WO2004/089381 (assigned to NexMed Holdings, Inc.) disclose topical compositions for transdermal delivery of prostaglandin E₁, wherein the PGE₁ is present in a non-derivatized form, which include a transdermal penetration enhancer, a polysaccharide or a polymer of polyacrylic acid and a lipophilic compound, in addition to a buffer system. It should be noted that the compositions are defined as "topical" since they are intended to act in the vicinity of the site where they are applied, even if they pass through the skin tissues. Specifically, PGE₁-based topical preparations proposed by the three cited patent documents are indicated for use in erectile dysfunction, female sexual dysfunction and in vasospasm, to increase the blood flow in tissues adjacent to the point of application of the preparation and then to treat problems of ischemic nature in limited areas of the body.

The International Patent Application publ. No. WO2008/041054 (on behalf of Techfields Biochem Co. Ltd) describes new water-soluble pro-drugs equipped with positive charge, which may be made from any prostaglandin or prostacyclin, in order to improve the capacity of transdermal permeation. The therapeutic indications described in this document for each of the various known active prostaglandins or prostacyclins are the indications that were already known at the filing date of the document in question. Although the concerned document refers in general to transdermal administration, for all ophthalmic pathologies the document only discloses a topical administration to the eye, and not an administration through the skin surface.
Akiharu Isowaki et al. describe the transdermal delivery of hydrophobic drugs to the eye. This transdermal delivery is more effective than topical or oral administration of the drugs, and can be used as a new treatment for ocular diseases (Akiharu Isowaki, et al., Drug delivery to the Eye with a Transdermal Therapeutic System. Biol. Pharm. Bull. 26(1) 69-72 (2003)).

### Summary of the invention

In the light of the foregoing prior art, the object of the present invention is, therefore, to provide a therapy for chronic optic neuropathies and retinopathies of ischemic nature or accompanying ischemic manifestations in the ocular district which allows to treat promptly and successfully these pathologies, without having to subject the patient to demanding measures such as the administration of drugs by the intravenous route.

According to the invention, it has been found that the administration of appropriate doses of prostaglandin E₁ (PGE₁) in any suitable vehicle for skin application, on a peripheral skin surface, and in particular on the upper limbs surface, in skin areas particularly suitable for the transdermal passage, is effective to achieve the desired anti-ischemic effect of PGE₁ in the ocular district.

In the frame of the studies connected with the present invention, the intravenous route of administration of PGE₁ in cases of ophthalmic pathologies with an ischemic basis has been preliminarily evaluated, considering the related effects. When used intravenously according to the proposals of the prior art, PGE₁ is administered in an amount of 1 g per kg of patient body weight within two or three hours. So, for example, one patient weighing 60 kg receives 60 micrograms of PGE₁, diluted in 100 or 250 ml of physiological solution for intravenous infusion.

Using PGE₁ prepared in a vehicle for skin application, particularly in the form of a cream with a concentration of 10-30 micrograms of PGE₁ per ml of cream, and applying the cream on the surface of the patient's arm where the skin is softer, namely on the inner forearm, it has been experimentally found an effect of increased blood flow at the ocular district level such as to be able to successfully treat ischemic eye diseases, without the need of administering the drug intravenously. Specifically, with a cream made of PGE₁ at a concentration of 20 µg per ml of cream, by administering to a patient weighing 60 kg a total dose of 4 ml of cream (80 g total of PGE₁) suitably applied to the skin for transdermal absorption, anti-ischemic effects can be obtained in the ocular district which are comparable to those obtained with the administration of 60 micrograms of PGE1 by intravenous infusion.

Further according to the present invention, similar anti-ischemic effects in the ocular district can be obtained by administration of an appropriate quantity and concentration of PGE₁ in a transdermal patch applied to the skin in a suitable peripheral zone, or in a preparation for administration through the mucous membranes of the nasal cavity.

### Detailed description of the invention

Thus, the present invention specifically provides a preparation containing prostaglandin E₁ (PGE₁) as active ingredient in a carrier suitable for administration on the skin or on mucous membranes, for use in the transdermal or transmucosal treatment of ischemic ophthalmic pathologies selected, from the group consisting of: chronic ischemic optic neuropathies and chronic ischemic retinopathies wherein said treatment is carried out by administration of said preparation on one or more peripheral areas of the skin surface, or by administration of said preparation in the nasal cavities.

As already noted, ophthalmic pathologies that may be advantageously treated by administration of the preparation according the invention are ophthalmic pathologies of chronic nature accompanying ischemic manifestations in the ocular district as, in particular, high myopia, macular degeneration, glaucoma and diabetic retinopathy.

According to the invention, the treatment by transdermal route of the cited ophthalmic pathologies may be carried out by applying the prostaglandin E₁ preparation on skin surface areas of the upper limbs. Said zones should be, preferably, substantially glabrous and with a thin stratum corneum, in order to enhance the permeation of the active ingredient. Preferably, the preparation will be applied on the skin of the forearms, on the inner side.

In case of administration of PGE₁ by the transdermal route, the proposed preparation can be in the form of a cream, emulsion, solution or suspension, spray, lotion, ointment, pomade, liposomes or gel, or it can be in the form of a suitable transdermal patch, in the matrix of which PGE₁ is present, possibly together with an agent enhancing transdermal penetration, in a concentration such as to achieve the desired release profile for the treatment of the ocular ischemic pathologies considered.

In case of administration of PGE1 by the transmucosal route, the proposed preparation, which must be dispensed on the mucous membranes of the nasal cavities, may be chosen, for example, from a nasal spray based on liposomes, a nasal spray in nanoemulsion and a nasal spray in cyclodextrins solution in. This preparation may be formulated according to known pharmaceutical technology, in such a way as to achieve, with the delivery of each spray of product, the desired systemic release profile for therapy of ischemic ocular pathologies considered.

In general, for the therapy by means of application on the skin surface of the upper limbs, the preparation containing prostaglandin E1 according to the invention may be in the form of cream, ointment, liniment, spray, lotion, liposomes or gel, each of these forms being achievable according to conventional formulation in the field of pharmaceutical technique. Preferably the preparation contains a concentration of 10 to 30 µg / ml of prostaglandin E₁ as the active ingredient, optionally in admixture with one or more agents promoters of transdermal permeation.

In particular, according to the invention, said transdermal treatment consists of repeated applying of the preparation in form of a cream on the skin of both forearms, on the inner side. Preferably, the cream has a concentration of active ingredient between 15 and 25 µg per ml of cream, preferably 20 ml per ml of cream. This concentration seems to be the most suitable one in view of the fact that, with a higher concentration, in case of overdose, undesired hypotensive effects might arise, and with a lower concentration the total time required for the transdermal absorption of the total dose required would stretch excessively.

According to some preferred embodiments of the present invention, the preparation of PGE1 is presented as a cream that the patient may apply himself following the instructions of the attending physician, and without the need to go each time in hospital. A special container allows the patient to apply to the skin a minimum dosage of 0.5 to 1.0 ml, which contains from 10 to 20 micrograms of PGE1.

In implementing the therapy, the patient applies from 10 to 20 g of product on the inside of the arm, in the area free from hair between the wrist and the elbow, where the skin is softer and therefore the absorption is easier. It is preferable to spread the cream using the same portion of the other forearm skin, always from the inside, by rubbing the two parts between them and distributing the cream in a uniform manner so as to cover all the skin inside of both arms.

The arms covered with the product should be exposed to air for at least 20 minutes (preferably from 30 to 45 minutes), the time required for complete absorption of the cream. It is necessary to prevent the product from coming in contact with clothing, as part of a product that ends up on your clothes is not absorbed by the skin.

After that the first quantity has been absorbed, another dose of cream, 0.5 to 1 ml, was applied following the same mode and waiting again at least 20 minutes to dry the cream. It goes on like this for 5-8 applications, depending on the physician's prescription, waiting each time that the drug is absorbed through the skin. The number of applications of each treatment is calculated by the physician based on the weight of the patient, so that the patient receives on the skin a total of 1,2 to 1,5 micrograms of PGE₁ per kg of weight over a two-or three hours.

It should be borne in mind that if the PGE₁ was administered more quickly side effects may occur such as hypotension. By hypotension, less blood reaches the eye and the patient may feel weak. In any case, a potassium oral intake before treatment may be foreseen, in order to prevent cardiac arrhythmias.

To improve the transdermal passage of the drug, the preparation according to the invention may contain among excipients, one or more active components such as transdermal penetration enhancers, chosen from those known and used for this purpose in the pharmaceutical art.

Based on the foregoing, a particularly preferred embodiment of the preparation of PGE₁ according to the invention is presented in the form of cream to be applied by the patient himself on the skin of both forearms, on the inner side, in quantities such as to contain 20 µg of prostaglandin E₁, allowed to dry on the skin for at least 20 min. (preferably 30-45 min.) and reapplied again in the same manner, repeating the operation until reaching the desired total dose.

Formulations particularly suitable for implementing the proposed treatment according to the invention are those in which the vehicle is constituted by a cream base of the type of those normally used in galenic preparations in aqueous base. These consist essentially of oil-in-water emulsions or aqueous dispersions of microcrystalline fatty acids and / or long-chain alcohols, with the addition of any optional excipients with specific functions, such as emulsifiers and co-emulsifiers, hydrophilic or lipophilic gelling agents, humectants and solvents, emollients, permeation enhancers agents or other secondary functional substances, such as acidifying agents, alkalizing, buffers, preservatives and antimicrobials, antioxidants, chelating agents, dyes, perfumes.

As fatty substances to be included in the formulation of the preparation of the invention may be used vegetable oils (such as almond oil, jojoba, avocado), mineral oils, oils of animal origin (such as lanolin), synthetic oils (such as esters isopropyl myristate, dodecyl oleate, isopropyl palmitate), silicone oils (such as dimethicone and cyclomethicone) and fluorinated oils. It is also possible to use alcohols and fatty acids, and waxes (such as beeswax and rice wax).

As emulsifiers and coemulsifiers may be used esters of polyglycerols and fatty acids, esters of sucrose and fatty acids, esters of sorbitan and fatty acids, esters of sorbitan and ethoxylated fatty acids, fatty alcohols and PEG ethers, the ethers of glycerol and fatty acids, the ackyl sulfates, the alkyl ethersulphate, the alkyl phosphates, alkyl polyglucosides, copolyols of dime-thico-ne. Examples of emulsifiers are ethoxylated steareth-2 and steareth-21-, while examples of glucoside-based emulsifiers the ceteraryl glucoside and the arakyl dilglucoside.

As hydrophilic gelling agents, are normally employed carboxyvinyl polymers (carbomer), acrylic copolymers such as copolymers of acrylates / alkyl acrylates, polyacrylamides, polysaccharides such as xanthan gum, the carrageenans, guar gum. As lipophilic gelling agents, may be used clays, metal salts of fatty acids, hydrophobic silica and ethylcellulose.

The agents with functions of humectants and solvents that may be included in the formulation, in addition to water, are for example glycerin, propylene glycol, butylene glycol, sorbitol, xylitol, betaine. The humectants are agents that typically promote moisture retention, for example moisturizing agents. Further examples of wetting agents include siloxanes, sorbitol, glycerin, glicereth-5-lactate, glicereth-7-triacetate, hexane triol, as methyl propane diol glycols, 1,2-pentane diol, hexylene glycol and propylene glycol, alkoxylated glucose, D-panthenol and its derivatives.

The emollient agents are compounds that soften and make smooth the epidermis, and may thus facilitate the passage of the product through the corneum stratum. Examples of emollients include oils and waxes such as microcrystalline wax, triglyceride esters such as castor oil, safflower oil, corn oil, olive oil, almond oil, palm oil, cocoa butter, squalene, acetylated monoglycerides, ethoxylated glycerides, fatty acids, alkyl esters of fatty acids, alkenyl esters of fatty acids, alcohols, acids, lanolin and its derivatives, esters of polyhydric alcohols, esters of waxes such as beeswax, vegetable waxes, isopropyl palmitate and glyceryl stearate.

As already noted, the transdermal passage of prostaglandin E1 can be increased by using in formulating compounds that promote the permeation of the active ingredient, such as dimethyl sulfoxide, taurocholates, membrane phospholipids and various surfactant agents suitable for use dermatological. Other components already used as transdermal penetration enhancers are alcohols or glycols (such as ethanol and propylene glycol), fatty acids (such as oleic acid) and their esters (such as dimethyl isosorbide).

Regarding any suitable buffers, acidifying or alkalizing agents that maintain or bring the pH of the composition in the field physiologically acceptable, as well as preservatives and antimicrobials, antioxidants, chelating agents, dyes and fragrances to be included in the formulation of PGE1 preparation for the 'therapeutic use according to the invention, the most suitable components may be selected from those known and used in the pharmaceutical art.

Exclusively by way of example, a possible formulation of cream suited as a vehicle for a preparation of PGE1 for use in the therapy of ischemic ophthalmic pathologies according to the present invention may have the following ingredients:
water, liquid paraffin, cetyl alcohol (C₁₆), polyoxyethylene stearate (25) (*emulsifier*), glycerol stearate (*emulsifier, surfacfant, emollient*), glycerin, stearyl alcohol (C₁₈), dimethicone PEG100 stearate (*emollient*), EDTA (*chelating agent*), methylparaben e propylparaben (*preservative*), butyl hydroxyanisole (BHA) (*antioxidant*), tocopherol (*anfioxidant*), triethylcitrate (*anfibacferial deodorant*), Transcutol (or diethylene glycol monoethylether, or carbitol) (*multi-functional addictive with functions of solubilizing the active ingredients, stratum corneum permeation enhancer, stabilizer for some molecules*).

Again by way of example, a possible formulation of trans-dermal patch with PGE₁ suitable for the treatment of ischemic ophthalmic pathologies according to the invention can be realized on the basis of Example 1 of the patent EP 0729538 (3M), already mentioned, by changing the superficial concentrations of active ingredient in the patch so as to achieve the desired release profile for the ophthalmic ischemic pathology to be treated.

Still by way of example, we report in the following three alternative formulations applicable for the transmucosal administration of PGE1, in the nasal cavities, suitable for the therapeutic purposes of the invention

Liposomal Nasal Spay: 0.5%phospholipids; 0.008% PGE₁, bihydrate bibasic sodium phosphate, monohydrate bibasic sodium phosphate, EDTA, NaCl, pH: 6.8-7.4, Osmolality 300 mOsm/kg, sterile for filtration 0.2 µm

Nasal Spray nanoemulsion: 0.5%phospholipids; 0.008% PGE₁, soybean oil 0.300 bihydrate bibasic sodium phosphate, monohydrate bibasic sodium phosphate, EDTA, glycerin pH: 6.8-7.4, Osmolality 300 mOsm/kg, sterile by filtration 0.2 µm

Nasal Spray solution in cyclodextrin: cyclodextrin 1.0%; PGE₁ 0.008%, bibasic sodium phosphate bihydrate, bibasic sodium phosphate monohydrate, EDTA, NaCl

pH: 6,8-7,4, Osmolality 300 mOsm/kg, sterile by filtration 0,2 µm.

### Examples

Some experimental results, obtained with the application of a specific embodiment of the present invention, including clinical data referring to the therapeutic results achieved with the application of the invention, are given by way of example in the following.

### Example 1 - Treatment of high myopia with transdermal administration of prostaglandin E₁

We studied eleven eyes in 6 patients with high myopia. The average of the axial length (which is usually between 22 and 24 mm) was 31.24 mm. Before being processed, all the subjects showed a visual field with an average deficit of -11.87 decibels (DB)

After two treatments according to the present invention, carried out on two different days, each with 80 micrograms of PGE₁ in cream with the previously described methods, the average deficit of the visual field was of -9.43 DB. The difference was statistically significant with a p-value = 0.02.

For more than a year, all subjects have continued the treatment with PGE cream with intervals which varied depending on the case, from 10 to 30 days. The therapeutic results obtained are summarized in the table below.

**Table 1**

| Patient | Age | Sex | Eye | Length (mm) | Visual field before (DB) | Visual field after (DB) |
|---|---|---|---|---|---|---|
| 1 | 57 | F | OD | 34.29 | -11.28 | -8.24 |
| | | | OS | 33.24 | -8.71 | -7.07 |
| 2 | 66 | F | OD | 30.37 | -5.72 | -5.66 |
| | | | OS | 30.36 | -5.27 | -4.61 |
| 3 | 71 | F | OD | 31.71 | -8.01 | -6.68 |
| | | | OS | 33.11 | -26.60 | -24.67 |
| 4 | 72 | M | OD | | | |
| | | | OS | 31.54 | -20.29 | -11.25 |
| 5 | 59 | M | OD | 33.10 | -7.13 | -6.51 |
| | | | OS | 32.22 | -8.16 | -6.23 |
| 6 | 69 | F | OD | 27.31 | -14.16 | -12.67 |
| | | | OS | 26.30 | -15.16 | -10.07 |

### Example 2 - Treatment of macular degeneration with transdermal administration of prostaglandin E1

Five patients with a macular degeneration were subjected to electrophysiologic examinations of the eye to measure the functionality of the retina. In particular they were subjected to multifocal electroretinogram (mfERG).

Subsequently the five patients were treated twice with 80 micrograms of PGE₁ cream in the previously described way. After one week they were again subjected to mfEGR and a clear improvement of the values has been found.

As noted above, ischemic heart disease is not the leading cause of macular degeneration, but contributes to the disease, and then the experiments carried out allows to conclude that the increase of blood flow in the ocular district improves the retinal function, as it is clear from the examination of mfERG , thereby relieving the symptoms of this serious chronic disease.

As is apparent from the foregoing, the application of PGE₁ in the form of a topical skin preparation with appropriate means and an appropriate dosage allows, in pathologies of the ocular district in which the treatment with PGE₁ would be indicated, to be able to avoid practicing an intravenous infusion of PGE₁ every 7-30 days. The patient may apply the cream or an equivalent transdermal patch autonomously remaining at home, and the need to be hospitalize every time in a medical-hospital structure for treatment is avoided.

The present invention has been disclosed with particular reference to some specific embodiments thereof, but it should be understood that modifications and changes may be made by the persons skilled in the art without departing from the scope of the invention as defined in the appended claims.

## Claims

1. A preparation containing prostaglandin E₁ as active ingredient in a carrier suitable for administration on the skin or on mucous membranes, for use in the transdermal or transmucosal treatment of ischemic ophthalmic pathologies selected from the group consisting of:
chronic ischemic optic neuropathies and chronic ischemic retinopathies wherein said treatment is carried out by administration of said preparation on one or more peripheral areas of the skin surface, or by administration of said preparation in the nasal cavities.

2. A preparation containing prostaglandin E₁ for the use according to claim 1, wherein said chronic ischemic optic neuropathies or said chronic ischemic retinopathies occur in high myopia, in macular degeneration, in glaucoma or in diabetic retinopathy.

3. A preparation containing prostaglandin E₁ for the use according to claims 1 or 2, wherein said transdermal treatment consists of applying said preparation on skin surface areas of the upper limbs.

4. A preparation containing prostaglandin E₁ for the use according to claim 3, wherein said preparation is in the form of a cream, an emulsion, a solution or a suspension, a spray, lotion, liniment, ointment, liposomes or a gel.

5. A preparation containing prostaglandin E₁ for the use according to claim 3, wherein said preparation is in the form of a transdermal patch.

6. A preparation containing prostaglandin E₁ for the use according to claims 1 or 2, wherein said transmucosal treatment consists of the administration of the preparation in the nasal cavity, and said preparation is selected from liposomal nasal sprays, microemulsion nasal sprays and cyclodextrin-solubilized nasal sprays.

7. A preparation containing prostaglandin E₁ for the use according to claims 1 or 2, wherein said preparation is in the form of a cream, ointment, liniment, liposomes or gel, and contains from 10 to 30 µg/ml of prostaglandin E₁ as active ingredient.

8. A preparation containing prostaglandin E₁ for the use according to claim 7, further containing one or more skin penetration enhancers.

9. A preparation containing prostaglandin E₁ for the use according to claim 7, wherein said transdermal treatment consists of repeatedly applying said preparation in cream on the skin of both forearms, on the internal side.

10. A preparation based on prostaglandin E₁ for the use according to claim 9, wherein in each application said preparation is applied in such amount as to contain 15-25 µg of prostaglandin E₁, and is allowed to dry on the skin.

11. A preparation based on prostaglandin E₁ for the use according to claim 10, wherein said preparation is applied more than once on the skin, with the same procedure, such operation being repeated until the desired total dose is reached.

## Patentansprüche

1. Präparation, die Prostaglandin E₁ als aktiven Bestandteil in einem Träger, der zur Verabreichung auf der Haut oder Schleimhautmembranen geeignet ist, enthält, zur Verwendung bei der transdermalen oder transmukosalen Behandlung von ischämischen ophthalmischen Krankheitsbildern, welche aus der Gruppe, die aus chronischen ischämischen Optikus-Neuropathien und chronischen ischämischen Retinopathien besteht, ausgewählt ist,
wobei die Behandlung durch Verabreichung der Präparation auf einem oder mehreren peripheren Bereich(en) der Hautoberfläche oder durch Verabreichung der Präparation in den Nasenhöhlen erfolgt.

2. Präparation, die Prostaglandin E₁ enthält, zur Verwendung nach Anspruch 1, wobei die chronischen ischämischen Optikus-Neuropathien oder chronischen ischämischen Retinopathien bei starker Myopie, bei Makula-Degeneration, bei Glaukomen oder bei diabetischer Retinopathie auftreten.

3. Präparation, die Prostaglandin E₁ enthält, zur Verwendung nach den Ansprüchen 1 oder 2, wobei die transdermale Behandlung in der Applikation der Präparation auf Hautoberflächen der oberen Gliedmaßen besteht.

4. Präparation, die Prostaglandin E₁ enthält, zur Verwendung nach Anspruch 3, wobei die Präparation in Form einer Creme, einer Emulsion, einer Lösung oder einer Suspension, eines Sprays, einer Lotion, eines Liniments, einer Salbe, eines Gels oder in Form von Liposomen vorliegt.

5. Präparation, die Prostaglandin E₁ enthält, zur Verwendung nach Anspruch 3, wobei die Präparation in Form eines transdermalen Pflasters vorliegt.

6. Präparation, die Prostaglandin E₁ enthält, zur Verwendung nach den Ansprüchen 1 oder 2, wobei die transmukosale Behandlung in der Verabreichung der Präparation in der Nasenhöhle besteht und die Präparation aus liposomalen Nasensprays, Mikroemulsions-Nasensprays und Cyclodextrinsolubilisierten Nasensprays ausgewählt ist.

7. Präparation, die Prostaglandin E₁ enthält, zur Verwendung nach den Ansprüchen 1 oder 2, wobei die Präparation in Form einer Creme, Salbe, eines Liniments, Gels oder in Form von Liposomen vorliegt und 10 bis 30 µg/ml Prostaglandin E₁ als aktiven Bestandteil enthält.

8. Präparation, die Prostaglandin E₁ enthält, zur Verwendung nach Anspruch 7, welche ferner einen oder mehrere Hautpenetrationsförderer enthält.

9. Präparation, die Prostaglandin E₁ enthält, zur Verwendung nach Anspruch 7, wobei die transdermale Behandlung aus der wiederholten Applikation der Präparation in einer Creme auf die Haut von beiden Vorderarmen auf der Innenseite besteht.

10. Präparation auf der Basis von Prostaglandin E₁ zur Verwendung nach Anspruch 9, wobei bei jeder Applikation die Präparation in einer solchen Menge, dass 15 bis 25 µg Prostaglandin E₁ enthalten sind, appliziert wird und auf der Haut trocknen gelassen wird.

11. Präparation auf der Basis von Prostaglandin E₁ zur Verwendung nach Anspruch 10, wobei die Präparation mehr als einmal mit der gleichen Prozedur auf die Haut appliziert wird, wobei der Vorgang wiederholt wird bis die gewünschte Gesamtdosis erreicht ist.

## Revendications

1. Préparation contenant comme principe actif de la prostaglandine E₁ dans un véhicule approprié pour l'administration sur la peau ou les membranes muqueuses, destinée à être utilisée dans le traitement transdermique ou par voie transmuqueuse de pathologies ophtalmiques ischémiques choisies dans le groupe constitué par :
les neuropathies optiques ischémiques chroniques et les rétinopathies ischémiques chroniques,
ledit traitement étant réalisé par administration de ladite préparation sur une ou plusieurs régions périphériques de la surface de la peau, ou par administration de ladite préparation dans les cavités nasales.

2. Préparation contenant de la prostaglandine E₁, destinée à être utilisée selon la revendication 1, où lesdites neuropathies optiques ischémiques chroniques ou lesdites rétinopathies ischémiques chroniques surviennent dans des cas de forte myopie, de dégénérescence maculaire, de glaucome ou de rétinopathie diabétique.

3. Préparation contenant de la prostaglandine E₁, destinée à être utilisée selon la revendication 1 ou 2, où ledit traitement transdermique consiste à appliquer ladite préparation sur des régions de la peau des membres supérieurs.

4. Préparation contenant de la prostaglandine E₁, destinée à être utilisée selon la revendication 3, ladite préparation étant sous la forme d'une crème, d'une émulsion, d'une solution ou d'une suspension, d'une pulvérisation, d'une lotion, d'un liniment, d'une pommade, de liposomes ou d'un gel.

5. Préparation contenant de la prostaglandine E₁, destinée à être utilisée selon la revendication 3, ladite préparation étant sous la forme d'un timbre transdermique.

6. Préparation contenant de la prostaglandine E₁, destinée à être utilisée selon la revendication 1 ou 2, où ledit traitement par voie transmuqueuse consiste à administrer la préparation dans la cavité nasale, et ladite préparation est choisie parmi les pulvérisations nasales liposomiques, les pulvérisations nasales en microémulsion et les pulvérisations nasales solubilisées à la cyclodextrine.

7. Préparation contenant de la prostaglandine E₁, destinée à être utilisée selon la revendication 1 ou 2, ladite préparation étant sous la forme d'une crème, d'une pommade, d'un liniment, de liposomes ou d'un gel, et contient de 10 à 30 µg/ml de prostaglandine E₁ en tant que principe actif.

8. Préparation contenant de la prostaglandine E₁, destinée à être utilisée selon la revendication 7, contenant en outre un ou plusieurs activateurs de pénétration cutanée.

9. Préparation contenant de la prostaglandine E₁, destinée à être utilisée selon la revendication 7, où ledit traitement transdermique consiste à appliquer de manière répétée ladite préparation en crème sur la peau des deux avant-bras, sur la partie intérieure.

10. Préparation à base de prostaglandine E₁, destinée à être utilisée selon la revendication 9, où à chaque application, ladite préparation est appliquée en une quantité telle qu'elle contient 15-25 µg de prostaglandine E₁, et est laissée sécher sur la peau.

11. Préparation à base de prostaglandine E₁, destinée à être utilisée selon la revendication 10, ladite préparation étant appliquée plus d'une fois sur la peau, selon le même mode opératoire, une telle opération étant répétée jusqu'à atteindre la dose totale souhaitée.
